Europäisches Patentamt

European Patent Office

Office européen des brevets

(19)

(11) Numéro de publication: **0 600 963 B1**

(12) **FASCICULE DE BREVET EUROPEEN**

(45) Date de publication du fascicule du brevet: **20.12.95** (51) Int. Cl.⁶: **A61B 3/032**, A61B 3/06

(21) Numéro de dépôt: **92917568.5**

(22) Date de dépôt: **24.07.92**

(86) Numéro de dépôt internationale :
**PCT/FR92/00739**

(87) Numéro de publication internationale :
**WO 93/02614 (18.02.93 93/05)**

(54) **MATERIEL DE TEST D'ACUITE VISUELLE ET/OU DE SENSIBILITE AUX CONTRASTES SPATIAUX CHEZ L'HOMME, DISPOSITIF ET PROCEDE DE FABRICATION CORRESPONDANTS**

(30) Priorité: **01.08.91 FR 9109857**

(43) Date de publication de la demande:
**15.06.94 Bulletin 94/24**

(45) Mention de la délivrance du brevet:
**20.12.95 Bulletin 95/51**

(84) Etats contractants désignés:
**AT BE CH DE DK ES FR GB GR IT LI LU MC NL SE**

(56) Documents cités:
**FR-A- 2 342 051
GB-A- 2 045 457
GB-A- 2 064 160
US-A- 4 615 594
US-A- 4 789 234**

(73) Titulaire: **OPSIA
Avenue de l'Europe,
Imm. Theogone Parc Technologique du Canal
F-31520 Ramonville-Saint-Agne (FR)**

(72) Inventeur: **PYNSON, Joel
11 bis rue Bouquières
31000 TOULOUSE (FR)**

(74) Mandataire: **Cabinet BARRE LAFORGUE & associés
95, rue des Amidonniers
F-31000 Toulouse (FR)**

EP 0 600 963 B1

Rank Xerox (UK) Business Services
(3.10/3.09/3.3.3)

EP 0 600 963 B1

## Description

La présente invention concerne un matériel de test d'acuité visuelle et/ou de la sensibilité visuelle aux contrastes spatiaux chez l'homme, ainsi qu'un dispositif correspondant et un procédé de fabrication d'un tel test.

Les contrastes perçus par l'homme peuvent être de deux types, à savoir : temporels ou spatiaux. Les contrastes temporels correspondent à des variations dans le temps du contraste, comme par exemple les variations de luminosité d'un gyrophare. Les contrastes spatiaux concernent quant à eux des variations du contraste indépendamment du temps, mais s'étendant dans l'espace, c'est-à-dire par exemple une scène comportant des zones de différents contrastes, comme des dégradés de couleurs ou différents niveaux de gris (allant par exemple du noir profond au gris à peine teinté).

La présente invention concerne uniquement un test de détermination de la sensibilité aux contrastes spatiaux, chez l'homme. Dans la suite de la description, il est ainsi entendu que les termes "test de contrastes" signifient en fait "tests des contrastes spatiaux".

Des études cliniques récentes ont montré qu'il existe un intérêt certain à déterminer même pour des sujets présentant une bonne acuité visuelle, leur sensibilité visuelle aux contrastes spatiaux. En effet, certaines pathologies oculaires comme les cataractes, les glaucomes, les kératopathies, etc... peuvent se dépister à un stade très précoce, à l'aide de test de contrastes, alors que la fonction d'acuité visuelle est encore peu ou pas atteinte. Dépistées assez tôt, de telles maladies peuvent se soigner avec plus d'efficacité. De même, cette étude de le sensibilité aux contrastes permet d'évaluer certaines techniques chirurgicales, comme des greffes de cornées, etc...

Il est déjà connu des dispositifs permettant d'étudier le sensibilité aux contrastes chez l'homme. Le brevet FR 2.342.051 (Acadia associates) décrit par exemple un dispositif de détermination de la sensibilité aux contrastes comprenant un ensemble de visée binoculaire, dans lequel un patient voit apparaître des diapositives comportant comme optotypes des lettres sur un fond blanc. En faisant varier la luminosité du fond, on crée des contrastes absolus différents pour l'ensemble des lettres portées par une diapositive. On détermine alors pour chaque taille de lettre (c'est-à-dire chaque fréquence spatiale de l'optotype) le contraste minimal perçu par le patient.

L'ensemble de ces mesures est ensuite reporté sur une courbe indiquant le minimum de contraste perçu par le patient en fonction de la fréquence spatiale. Cette courbe est ensuite comparée à une courbe type établie pour un sujet sain. Les éventuelles variations de la courbe du patient par rapport à la courbe type permettent de mettre en évidence des pathologies oculaires...

Un des inconvénients majeurs d'un tel dispositif est qu'il nécessite un temps d'examen prolongé, notamment dû à la réalisation de courbes et à leur analyse. Un tel dispositif est bien trop long et complexe à mettre en oeuvre pour pouvoir être utilisé de manière systèmatique, au cours d'un examen ophtamologique classique de dépistage. En outre, un tel test déroute le patient et il est parfois difficile de lui faire comprendre son fonctionnement, ce qui nuit bien sûr à la qualité de l'examen mené.

Il est également déjà connu des dispositifs du type de celui décrit dans le brevet US 4.365.873 (Ginsburg) qui se présente sous la forme d'une feuille transparente comportant une pluralité de zones de test réparties sous forme de lignes et de colonnes. Chaque colonne présente des zones test de mêmes contrastes et chaque ligne des zones test de même fréquence spatiale.

Les motifs des zones de test sont réalisés par des réseaux, c'est-à-dire une alternance de zones sombres et claires de contrastes et de fréquences spatiales déterminées. Ces zones sombres et claires peuvent être de formes sinusoïdales ou carrées et sont soit verticales soit inclinées. Le feuille transparente est placée sur une face diffusante d'un caisson lumineux. Le patient est placé à une certaine distance du caisson et doit indiquer pour chacune des zones de test vues si son réseau est vertical ou incliné.

Le praticien note alors le niveau minimum de contraste discerné en fonction de la fréquence spatiale de la zone test discernée. Il est nécessaire ensuite de tracer pour chaque patient sa courbe de sensibilité aux contrastes en fonction des fréquences spatiales. Un tel dispositif est également fort long à mettre en place. En outre, le patient n'est pas très familier avec les réseaux présentés dans les zones de test. D'où, un risque de non compréhension du test qui peut entraîner une mesure complètement erronée de la sensibilité aux contrastes spatiaux.

En outre, les dispositifs connus à ce jour ne permettent pas de déterminer la sensibilité aux contrastes d'un patient, dans des conditions autres que celles d'un éclairage moyen de l'ordre de 80 à 90 cd/m$^2$. Or, certains patients se plaignent de troubles apparaissent uniquement dans des conditions spécifiques d'éclairement, à savoir, au crépuscule ou niveau mésopique (3 à 10 cd/m$^2$) ou par journée fortement ensoleillée (niveau photopique haut de l'ordre de 600 à 1 000 cd/m$^2$), voire lors d'un éblouissement (au-delà de 1 000 cd/m$^2$).

2

La présente invention a pour but de pallier l'ensemble de ces inconvénients et propose un matériel de test de contrastes facile ne déroutant pas le patient, rapide à effectuer et permettant la détermination immédiate de le fonction de sensibilité aux contrastes d'un patient en fonction de la fréquence spatiale, afin de mettre en évidence d'éventuelles pathologies ou autres anomalies oculaires.

En outre, le test de contraste selon l'invention peut également être utilisé comme un simple test d'acuité visuelle.

Un autre objectif de la présente invention est de pouvoir réaliser un test de sensibilité aux contrastes spatiaux chez l'homme et ceci dans différentes conditions d'éclairement, à savoir, à un niveau dit crépusculaire, à un niveau moyen et à un niveau correspondent à un fort ensoleillement, voire à un niveau d'éblouissement.

A cet effet, le présente invention concerne un matériel de test d'acuité visuelle et/ou de la sensibilité visuelle aux contrastes spatiaux chez l'homme selon la revendication 1, du type comportant une feuille munie sur l'une de ses faces d'une pluralité d'optotypes répartis sous forme de lignes et de colonnes, caractérisé en ce que les optotypes perçus par l'homme comme ayant une même valeur de contraste sont rangés selon une même colonne ou respectivement ligne, et d'autre part, les optotypes de même fréquence spatiale en correspondance avec celle utilisée pour des tests d'acuité sont rangés selon une même ligne ou respectivement colonne.

Ainsi, le matériel de test de contraste selon l'invention se présente sous la forme d'une simple feuille munie d'optotypes répartis sous forme de lignes et de colonnes. Il ressemble donc aux tests d'acuité visuelle classiques et ne déroute pas le patient.

Chaque colonne d'optotypes présente une valeur de contraste perçu par l'oeil humain comme unique et décroissante d'une colonne à l'autre, en partant d'un contraste maximum pour aller jusqu'au contraste minimum perçu par un sujet sain.

En outre, chacune des lignes présente une même fréquence spatiale.

Avantageusement, les fréquences spatiales testées correspondent directement à celles utilisées pour les tests d'acuité visuelle traditionnels. Ainsi, le praticien établit immédiatement et sans avoir à dessiner de courbe spécifique les performances de son patient en ce qui concerne sa sensibilité aux contrastes spatiaux en fonction de chaque fréquence spatiale correspondent à un degré d'acuité visuelle. Ces performances se lisent en fait directement sur la feuille portant les optotypes.

Réciproquement, la fonction des lignes et des colonnes peut être inversée. Ainsi, chaque ligne peut présenter des optotypes de même contraste perçu et chaque colonne peut présenter des optotypes de même fréquence spatiale.

De préférence, les optotypes utilisés sont des lettres. De ce fait, le patient a tendance à se prêter sans difficulté à ce nouvel examen qu'il comprend facilement en raison de sa similitude avec les tests d'acuité traditionnels.

Avantageusement, un tel test est porté par une feuille transparente ou translucide à placer sur la face diffusante d'un caisson lumineux.

Selon une variante de réalisation, les optotypes représentés sont des images ou symboles facilement reconnaissables par des enfants ou des illettrés.

La présente invention a également pour but de créer un dispositif mettant en oeuvre le matériel de test d'acuité et/ou de contrastes ci-dessus décrit.

L'objectif d'un tel dispositif est de permettre d'effectuer un test de contrastes d'une manière fiable et reproductible et sous différentes conditions de luminosité. Il s'agit ici de dépister toute anomalie dans la perception des contrastes, aussi bien sous luminosité normale, qu'en luminosité crépusculaire, ou ensoleillée, voire sous éblouissement.

A cet effet, la présente invention concerne un dispositif de détermination de l'acuité visuelle et/ou de la sensibilité aux contrastes chez l'homme selon la revendication 11, du type comportent un matériel de test d'acuité et/ou de contrastes, caractérisé en ce qu'il comporte en combinaison :

- un moyen de support du matériel de test d'acuité et/ou de contrastes, muni d'une face frontale diffusante adaptée pour porter ledit test,
- un moyen d'éclairement logé à l'intérieur du moyen de support et adapté pour éclairer uniformément selon une luminosité prédéterminée la face frontale du moyen de support et,
- la feuille dudit matériel de test d'acuité et/ou de contrastas placée sur la face frontale du moyen de support, ladite feuille comportant une pluralité de lignes et de colonnes d'optotypes, les optotypes perçus par l'homme comme ayant une même valeur de contraste sont rangés selon une même colonne ou respectivement ligne, et, d'autre part, les optotypes de même fréquence spatiale en correspondance avec celle utilisée pour des tests d'acuité sont rangés selon une même ligne ou respectivement colonne.

Un tel dispositif présente ainsi un moyen de support pour le matériel de test de contraste. Un moyen d'éclairement situé à l'intérieur du support permet d'éclairer uniformément une face diffusante du moyen de support, sur laquelle est placé le matériel de test d'acuité et/ou de contrastes. L'éclairement résultant est prédéterminé et permet de mettre en oeuvre le test selon l'invention sous des conditions de luminosité contrôlées.

De préférence, un tel dispositif est muni de moyens de sélection de l'éclairement adapté pour permettre un choix des conditions de luminosité sous lesquelles le test est réalisé. Ces moyens de sélection commandent l'intensité délivrée à la source lumineuse.

De préférence, la source lumineuse comprend une pluralité de tubes-néons.

Une telle source lumineuse est adaptée pour éclairer uniformément l'ensemble de la face diffusante du moyen de support portent le matériel de test. On obtient ainsi des éclairements de la face diffusante, de l'ordre de 3 à 10 cd/m$^2$ (crépuscule), 80 à 90 cd/m$^2$ (journée normale), 600 à 1 000 cd/m$^2$, voire plus (journée fortement éclairée).

La présente invention a également pour objectif le création d'un procédé de réalisation du test selon la revendication 22.

Un tel procédé a pour but de créer un test d'acuité visuelle et/ou de sensibilité eux contrastes spatiaux chez l'homme, dans lequel chaque optotype présente un contraste strictement défini, vérifiable et ajustable. En effet, étant donné les faibles contrastes de certains optotypes du matériel de test selon l'invention, il est impératif pour que le matériel de test soit fiable et apporte une information réelle, que les tests fabriqués soient strictement définis.

En outre, il s'agit d'éviter toute dégredation dans le temps du test, car cela nuirait à un examen correct du patient.

A cet effet, la présente invention concerne un procédé de fabrication d'un matériel de test d'acuité visuelle et/ou de sensibilité aux contrastes, caractérisé en ce qu'il consiste à :

- créer à l'aide d'une unité centrale de calcul des optotypes de grandeur déterminée afin de leur conférer une fréquence spatiale spécifique,
- donner aux optotypes de grandeur déterminée une densité déterminée afin de leur conférer une valeur de contraste spécifique,
- ranger tous les optotypes de même fréquence spatiale selon une même ligne ou respectivement selon une même colonne,
- ranger tous les optotypes perçus par l'homme comme ayant les mêmes contrastes selon une même colonne ou respectivement selon une même ligne,
- afficher sur un écran associé à l'unité centrale le tableau d'optotypes ainsi créés,
- réaliser un positif de l'écran sur un film photosensible,
- réaliser un négatif du positif,
- tirer sur une feuille ledit négatif,
- vérifier le contraste de chacun des optotypes apparaissent sur le tirage,
- en déduire les temps d'exposition optimum du négatif pour obtenir les contrastes désirés et,
- tirer ledit négatif afin de réaliser une pluralité de matériel de tests d'acuité visuelle et/ou de sensibilité aux contrastes identiques.

Un tel procédé de fabrication permet de contrôler rigoureusement le contraste de chaque optotype créé sur le négatif. De ce fait, en modifiant les temps d'exposition à la lumière, du négatif, il est possible d'obtenir pour le tirage finel des valeurs de contrastes précisément définies, et reproductibles. Ainsi, le matériel de test selon l'invention présente des optotypes définis avec précision et une qualité identique pour l'ensemble des matériaux de test réalisés. En outre, un tel matériel de test n'a pas tendance à se détériorer dans le temps sous l'action de la lumière.

D'autres objets, caractéristiques ou avantages de le présente invention ressortiront de le description qui suit en référence aux dessins annexés, dans lesquels :

- la figure 1 est une vue en perspective d'un dispositif de détermination de l'acuité visuelle et/ou de la sensibilité aux contrastes muni d'un matériel de test selon l'invention,
- la figure 2 est une vue schématique en coupe selon la ligne II-II de la figure 1,
- la figure 3 est une vue en coupe transversale du dispositif selon l'invention, et
- la figure 4 est un exemple de réalisation selon une première variante du matériel de test selon l'invention.

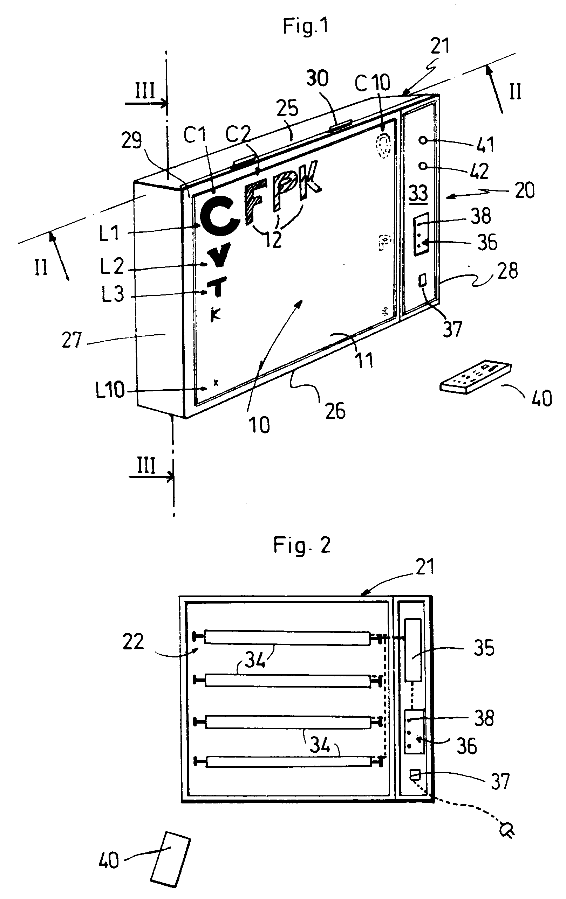

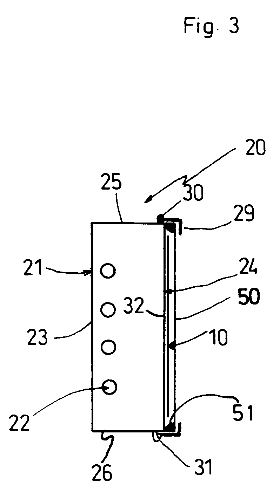

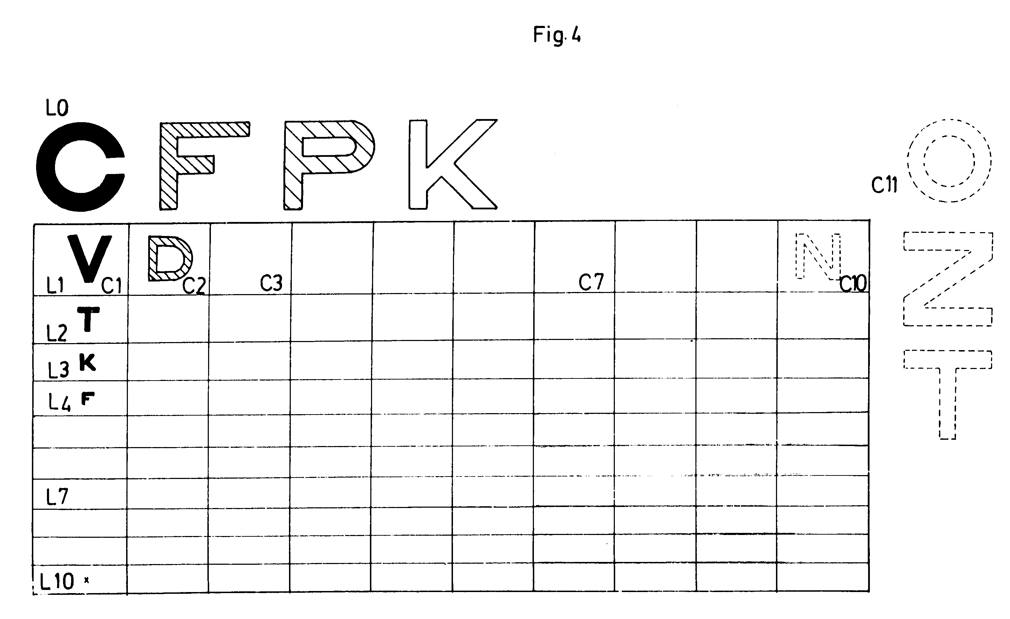

Selon la forme de réalisation représentée aux figures 1 et 3, le matériel 10 de test d'acuité visuelle et/ou de sensibilité aux contrastes spatiaux selon l'invention, comprend une feuille 11 en matière translucide munie sur une de ses faces d'une pluralité d'optotypes 12. Ces optotypes sont constitués, en l'exemple représenté, par des lettres. Le matériel de test comporte ainsi une pluralité de lignes $L_1$ à $L_{10}$ et une

4

pluralité de colonnes $C_1$ à $C_{10}$. Sur les figures 1 et 4, seule une partie des optotypes a été représentée pour ne pas obscurcir les dessins. En fait, à chaque intersection d'une ligne et d'une colonne on trouve un optotype.

Chaque colonne présente des optotypes perçus par l'oeil humain comme ayant un même contraste. Le première colonne $C_1$ présente un contraste perçu comme maximum, et chaque colonne suivante présente un contraste perçu comme décroissant pour arriver à le dernière colonne $C_{10}$ dont le contraste perçu est minimum. Ce contraste minimum perçu correspond au contraste minimum perçu par un sujet sain. Cette valeur de ce contraste a été obtenu par des tests cliniques. La décroissance des contrastes entre les colonnes $C_1$ et $C_{10}$ est logarithmique, afin de suivre le perception naturelle des contrastes chez l'homme.

Le contraste à l'intérieur d'une même colonne n'est pas strictement identique pour chacun des optotypes, même s'il est perçu par l'oeil humain comme identique. En effet, le contraste de chacun des optotypes d'une même colonne est déterminé de sorte que l'oeil humain le perçoive comme virtuellement identique, mais chacune des valeurs de contrastes dans une même colonne est différente.

Pour déterminer les valeurs réelles de contrastes, il faut tenir compte du fait que la valeur de contraste d'un optotype dépend de sa taille, c'est-à-dire de sa fréquence spatiale. Pour chaque lettre utilisée il faut en outre tenir compte de la taille du détail (dans cette lettre) qui permet de reconnaître la lettre. Donc pour chaque lettre, et en fonction de la taille du détail de reconnaissance de cette lettre, on est amené à déterminer une densité telle (un contraste) que l'oeil humain perçoive comme étant de contrastes identiques deux lettres dont les tailles diffèrent et dont le contraste réel en terme de densité sera en fait différent.

On définit de telles colonnes comme présentant une isosensibilité aux contrastes spatiaux chez l'homme. Le matériel de test selon l'invention présente ainsi dix colonnes d'isosensibilité aux contrastes spatiaux. Dans chacune de ces colonnes, la valeur de contraste des optotypes suit en outre la courbe de sensibilité eux contrastes chez l'homme, ce qui permet à l'oeil humain de les percevoir comme présentant un même contraste.

Ainsi, la colonne de plus faible contraste perçu présente comme valeurs de contraste de chacun des optotypes qui la compose de la première à la dernière ligne les valeurs suivantes : pour les lignes $L_1$ et $L_2$ le contraste est de 2,0 à 3,0 % par rapport au fond sur lequel sont inscrites les lettres, pour les lignes $L_3$ et $L_4$, 3,1 à 3,5 %, pour les lignes $L_5$ et $L_6$, 3,6 à 4,0 %, pour les lignes $L_7$ et $L_8$, 4,1 à 5,0 % et pour les lignes $L_9$ et $L_{10}$, 5,1 à 6,5 %.

Les colonnes précédentes augmentent de façon logarithmique chacune de ces valeurs de contrastes. On trouvera ci-après un tableau I récapitulant pour chaque colonne 1 à 10 et chaque ligne 1 à 10 le contraste des optotypes exprimé en pourcentage par rapport au fond, c'est-à-dire eu reste de la feuille supportent les optotypes.

TABLEAU I

| Colonne / Ligne | 1 | 2 | 3 | 4 | 5 | 6 | 7 | 8 | 9 | 10 |
|---|---|---|---|---|---|---|---|---|---|---|
| 1 et 2 | 95-100 | 26-30 | 18-21 | 12-15 | 7-10 | 5,1-6,5 | 4,1-5,0 | 3,6-4,0 | 3,1-3,5 | 2,0-3,0 |
| 3 et 4 | 95-100 | 32-36 | 22-25 | 18-21 | 12-15 | 7-10 | 5,1-6,5 | 4,1-5,0 | 3,6-4,0 | 3,1-3,5 |
| 5 et 6 | 95-100 | 38-42 | 26-30 | 22-25 | 18-21 | 12-15 | 7-10 | 5,1-6,5 | 4,1-5,0 | 3,6-4,0 |
| 7 et 8 | 95-100 | 44-48 | 31-36 | 26-30 | 22-25 | 18-21 | 12-15 | 7-10 | 5,1-6,5 | 4,1-5,0 |
| 9 et 10 | 95-100 | 50-55 | 38-42 | 32-36 | 26-30 | 22-23 | 18-21 | 12-15 | 7-10 | 5,1-6,5 |

Les lignes $L_1$ à $L_{10}$ présentent quant à elles des optotypes de fréquence spatiale déterminées et avantageusement en correspondance avec les fréquences spatiales utilisées pour les tests d'acuité.

Ainsi, les fréquences spatiales croissent de la première $L_1$ à la dernière ligne $L_{10}$ et sont les suivantes : 3 ; 6 ; 9 ; 12 ; 15 ; 17,5 ; 20 ; 24 ; 27 et 30 cycles par degré. Ces fréquences spatiales correspondent respectivement à celles utilisées pour la détermination des degrés d'acuité suivants : 1/10 ; 2/10 ; 3/10 ; 4/10 ; 5/10 ; 6/10 ; 7/10 ; 8/10 ; 9/10 et 1.

Il est rappelé que la fréquence spatiale d'un optotype est définie comme étant l'inverse de la dimension du détail de l'optotype exprimé en degrés (puisqu'il est vu à une certaine distance et sous un certain angle) et est exprimée en cycles par degré.

Le matériel ainsi décrit est destiné à être mis en place sur un dispositif 20 de détermination de l'acuité visuelle et/ou de la sensibilité visuelle aux contrastes spatiaux chez l'homme décrit ci-après.

Ce dispositif comporte notamment un moyen de support 21, un moyen d'éclairement 22 et le matériel de test 10.

Le moyen de support 21 (figure 3) est constitué par un caisson parallélépipédique comportant une face dorsale 23, une face frontale 24, des faces supérieure et inférieure 25, 26 et deux faces latérales 27 et 28 (figure 1). Un couvercle 29 vient coiffer le pourtour de la face frontale 24 du caisson. Ce couvercle est fixé à la face supérieure du caisson par une charnière 30 et est maintenu contre la face inférieure 26 du caisson par un dispositif de retenu 31.

La face frontale 24 du caisson 21 est séparée en deux zones distinctes, une première partie étant constituée par une plaque diffusante 32 (figure 3) et une seconde partie par un tableau de commande 33 (figure 1). Le matériel de test selon l'invention est placé contre la partie diffusante 32 (figure 3) de sorte que les optotypes qu'il comporte soient lisibles de l'extérieur du caisson. Une plaque en verre traité anti-reflet 50 est plaquée contre la face diffusante 32 de sorte que le matériel de test soit pris entre la plaque diffusante 32 et le verre traité 50. Un cordon de silicone 51 est placé tout autour de la plaque de verre 32 afin d'adhérer à chacune de ces deux plaques 32 et 50. Ce cordon 51 s'étend ainsi sur toute la périphérie des plaques, sauf en un point par lequel on aspire l'air emprisonné entre les plaques 32 et 50, de sorte qu'un vide soit créé entre ces deux plaques. Le joint de silicone est ensuite refermé. Un tel joint assure d'une part, qu'aucune bulle d'air n'est présente entre les plaques 32 et 50 et d'autre part qu'aucune poussière ou pollution puisse s'introduire entre ces deux plaques 32 et 50. On garantit ainsi une bonne résistance au vieillissement de l'ensemble.

Le moyen d'éclairement 22 est logé à l'intérieur du caisson et est constitué par une source lumineuse comportant au moins quatre tubes-néons 34.

Ces tubes-néons sont alimentés en énergie par un ensemble électronique 35, lui-même commandé par un moyen de sélection 36. Ce moyen de sélection est muni notamment de trois boutons de sélection 38. Une alimentation en énergie fournit l'énergie nécessaire au moyen de sélection et à l'ensemble électronique pour fonctionner. Cette alimentation est munie d'un interrupteur 37 de marche/arrêt. Le fonctionnement de ce dispositif est décrit ci-après.

Une fois l'interrupteur 37 mis sur la position marche, les moyens de sélection et l'ensemble électronique sont alimentés en énergie. Le praticien appuie sur l'un des boutons 38 de sélection. Le moyen de sélection envoie alors un signal de sélection à l'ensemble électronique lui imposant d'alimenter les tubes-néons à une certaine énergie, de sorte que le moyen d'éclairement éclaire la face diffusante 24 selon une certaine luminosité pré-établie.

De préférence, le moyen de sélection présente trois choix de luminosité possible, chacun associé à un des boutons 38. Un premier niveau de luminosité, dit niveau mésopique, provoque un éclairement de la face diffusante du caisson à une valeur de l'ordre de 3 à 10 cd/m². Un tel éclairement correspond à celui d'un crépuscule.

Un second niveau de luminosité correspond au niveau moyen d'éclairement d'une journée sans soleil direct, et provoque un éclairement de la face diffusante 32 de l'ordre de 80 à 90 cd/m².

Un troisième niveau de luminosité correspond à l'éclairement d'une journée fortement ensoleillée et provoque un éclairement de la face diffusante du caisson de 600 à 1 000 cd/m².

Le moyen de sélection comporte en outre un dispositif de télécommande à distance 40, permettant d'envoyer, par rayonnement infrarouge vers un récepteur infrarouge 41 associé à l'ensemble électronique, le signal de sélection de le luminosité choisie.

Le tableau de commande 33 est en outre muni d'un voyant de mise sous tension 42.

Lorsque le praticien a choisi l'éclairement de la face diffusante, soit directement par le moyen de sélection 36, soit par télécommande infrarouge, le moyen d'éclairement éclaire uniformément la plaque diffusante selon le niveau d'éclairement choisi.

Le praticien fait alors lire les lettres apparaissent sur la face diffusante à son patient. Il peut, à cet effet, utiliser un pointeur laser pour indiquer à son patient quelles sont les lettres à lire. Si nécessaire, ce test peut être effectué avec les autres valeurs de luminosité. Bien entendu, les contrastes minimaux perçus par un sujet sain dépendent de la luminosité de la face diffusante. Pour une luminosité de 80 à 90 cd/m$^2$, c'est la dernière colonne du matériel de test qui regroupe les contrastes minimaux perçus par un sujet sain. Pour les autres valeurs de luminosité de la face diffusante, ces valeurs minimales sont repérées sur chaque ligne du matériel de test. Une fois le test terminé, le praticien coupe l'alimentation du dispositif.

Il est à noter, bien sûr qu'aucune courbe ou analyse supplémentaire n'est nécessaire par le praticien pour établir un diagnostic concernant une éventuelle pathologie oculaire.

Un tel dispositif permet donc un dépistage aisé, rapide et systématique de toute anomalie de la perception des contrastes spatiaux, et ceci parce que la dernière colonne $C_{10}$ donne immédiatement le contraste minimum perçu par un sujet sain en fonction de chaque fréquence spatiale. Ainsi, si le patient arrive à lire la dernière colonne, le praticien sait immédiatement que sa sensibilité aux contrastes n'est pas perturbée. Si par contre le patient ne peut lire que jusqu'à moins une ou moins deux colonnes de $C_{10}$ c'est que sa sensibilité eux contrastes est diminuée. Le praticien sait en outre immédiatement que la sensiblité au contraste de ce patient est $C_9$ ou $C_8$. Aucune courbe n'est nécessaire pour étudier les performances de chaque patient par rapport aux performances d'un sujet sain.

Il est à noter que la simple lecture de le colonne à plus fort contraste permet de réaliser un test d'acuité traditionnel. Un tel matériel de test permet donc de déterminer l'acuité visuelle et/ou la sensiblité eux contrastes spatiaux.

En variante, le dispositif selon l'invention est muni de plus de quatre tubes-néons afin d'obtenir des éclairements plus importants, voir d'effectuer ce même test de sensibilité aux contrastes en état d'éblouissement.

En variante également, le matériel de test selon l'invention peut présenter toutes sortes d'optotypes, lettres, chiffres, symboles, images, ceci notamment pour effectuer un test de sensibilité aux contrastes spatiaux sur des enfants et des illettrés.

En variante encore et comme représenté à la figure 4, il peut s'avérer nécessaire de réaliser un test de contrastes pour une acuité visuelle inférieure à 1/10 et notamment pour une acuité de 1/20. Dans ce cas là, les optotypes correspondants sont plus grands que ceux de la ligne 1/10 et ne tiennent pas sur une ligne du test.

Selon cette variante de réalisation, cette ligne supplémentaire est partagée en une première ligne L0 et une onzième colonne entourant les optotypes du matériel de test de contraste précédemment décrits. Ceci permet d'utiliser avantageusement l'espace disponible pour le matériel de test sens l'augmenter pour ajouter cette ligne supplémentaire parfois nécessaire. De cette façon on n'augmente ni les dimensions du matériel, ni son poids, ni son coût.

On notera également que dans certains pays, certaines acuités intermédiaires (comme 1,5/10) ou supérieures (comme 12/10) sont testées. Dans ce cas, une ligne dont la fréquence spatiale correspond à cette acuité est ajoutée au tableau d'optotypes.

Le procédé de réalisation du matériel selon l'invention est décrit ci-après.

Pour réaliser le matériel selon l'invention :

- on crée à l'aide d'une unité centrale de calcul des optotypes de grandeur déterminée afin de leur conférer une fréquence spatiale spécifique,
- on donne aux optotypes de grandeur déterminée une densité déterminée afin de leur conférer une valeur de contraste spécifique,
- on range tous les optotypes de même fréquence spatiale selon une même ligne ou respectivement selon une même colonne,
- on range tous les optotypes perçus par l'homme comme ayant les mêmes contrastes selon une même colonne ou respectivement selon une même ligne,
- on affiche sur un écran associé à l'unité centrale le tableau d'optotypes ainsi créés,
- on réalise un positif de l'écran sur un film photosensible,
- on réalise un négatif du positif,
- on tire par un procédé photographique ledit négatif,
- on vérifie le contraste de chacun des optotypes apparaissant sur le tirage,
- on en déduit les temps d'exposition optimum du négatif pour obtenir les contrastes désirés et,
- on tire par un procédé photographique ledit négatif afin de réaliser une pluralité de matériels de tests d'acuité visuelle et/ou de contrastes identiques.

Un tel procédé de fabrication permet de contrôler rigoureusement la réalisation du matériel de test selon l'invention.

On garantit au matériel de test ainsi fabriqué une homogénéité et une qualité (notamment en ce qui concerne les valeurs de contrastes de chaque optotype).

Avantageusement, le tirage final est réalisé sur un film transparent ou translucide, on évite tout phénomène de vieillissement tel qu'il apparaît notamment avec le positif réalisé.

On notera cependant que le matériel de test selon l'invention peut également être réalisé sur une feuille opaque.

Bien entendu, la présente invention ne se limite pas au mode de réalisation ci-dessus décrit et englobe toute variante à la portée de l'homme de l'art.

Ainsi, le matériel de test selon l'invention peut être réalisé en noir et blanc ou en couleurs.

En outre, la rôle des lignes et des colonnes du test selon l'invention peut être inversé sans pour cela sortir du domaine de l'invention. Ainsi, il est tout à fait possible de créer des lignes d'isosensibilité aux contrastes spatiaux, et des colonnes ayant chacune une même fréquence spatiale.

De même, le matériel de test de contraste selon l'invention peut être réalisé sur un film qui est ensuite projeté sur une surface dont le luminosité est contrôlée.

Les valeurs de fréquences spatiales explorées par le test de contraste, selon l'invention, peuvent être autres que celles précédemment indiquées. De même, les valeurs de contrastes perçus explorées peuvent comporter des ensembles d'optotypes dont le contraste perçu est inférieur au minimum perçu par un sujet sain.

Il n'est pas nécessaire non plus de réaliser le présent test avec 10 lignes et 10 colonnes. En fait, ce test peut comporter un nombre quelconque et distinct respectivement de lignes et de colonnes.

De même, la source lumineuse utilisée dans le dispositif selon l'invention peut être constituée d'un seul tube-néon (ou de plusieurs) serpentant à l'intérieur du caisson et non plus rectiligne.

En outre, cette source lumineuse peut être réalisée avec des tubes fluorescents ou d'autres moyens équivalents. Le but ici est de réaliser un éclairement uniforme de la face diffusante 32.

## Revendications

1.  Matériel de test d'acuité visuelle et/ou de la sensibilité visuelle aux contrastes spatiaux chez l'homme, du type comportant une feuille munie sur l'une de ses faces d'une pluralité d'optotypes répartis sous forme de lignes et de colonnes, caractérisé en ce que :

    .   les optotypes d'une même fréquence spatiale sont rangés selon une même ligne $L_1$... $L_{10}$, ou respectivement colonne, ladite fréquence spatiale croissant de la première ligne $L_1$ à la dernière ligne $L_{10}$, en correspondance avec la fréquence spatiale utilisée pour des tests d'acuité,

    .   les optotypes sont rangés selon des colonnes $C_1$-$C_{10}$, ou respectivement des lignes, constituant des ensembles d'isosensibilité aux contrastes, et dont le contraste de luminance présente des valeurs décroissant d'une première colonne $C_1$, ou respectivement ligne, présentant une valeur de contraste maximale, jusqu'à une dernière colonne $C_{10}$, ou respectivement ligne, présentant une valeur minimale de contraste.

2.  Matériel de test selon la revendication 1, caractérisé en ce que la dernière colonne $C_{10}$ présente les valeurs de contrastes de luminance suivantes, exprimées en pourcentage de contrastes par rapport au fond sur lequel les optotypes sont réalisés : pour les lignes $L_1$ et $L_2$ un contraste de 2,0 à 3,0 %, pour les lignes $L_3$ et $L_4$, 3,1 à 3,5 %, pour les lignes $L_5$ et $L_6$, 3,6 à 4,0 %, pour les lignes $L_7$ et $L_8$, 4,1 à 5,0 % et pour les lignes $L_9$ et $L_{10}$, 5,1 à 6,5 %.

3.  Matériel de test selon l'une des revendications 1 ou 2, caractérisé en ce que les valeurs des contrastes sont décroissantes de façon logarithmique d'une colonne à la suivante.

4.  Matériel de test selon l'une des revendications 1 à 3, caractérisé en ce qu'il comporte 10 lignes et 10 colonnes.

5.  Matériel de test selon l'une des revendications 1 à 4, caractérisé en ce que la fréquence spatiale de chacune des lignes $L_1$ à $L_{10}$ correspond respectivement aux valeurs suivantes : 3 ; 6 ; 9 ; 12 ; 15 ; 17,5 ; 20 ; 24 ; 27 et 30 cycles par degré.

6.  Matériel de test selon l'une des revendications précédentes, caractérisé en ce que chacune des lignes $L_1$ à $L_{10}$ correspond, regardée à une distance prédéterminée, respectivement aux degrés d'acuité visuelle suivants : 1/10 ; 2/10 ; 3/10 ; 4/10 ; 5/10 ; 6/10 ; 7/10 ; 8/10 ; 9/10 ; 1.

7. Matériel de test selon l'une des revendications précédentes, caractérisé en ce que les optotypes utilisés sont des lettres.

8. Matériel de test selon l'une des revendications 1 à 6, caractérisé en ce que les optotypes utilisés sont des images ou des chiffres conçus pour pouvoir être reconnus par des enfants ou des illettrés.

9. Matériel de test selon l'une des revendications précédentes, caractérisé en ce que la feuille est translucide.

10. Matériel de test selon l'une des revendications 1, 2, 3, 7, 8, 9, caractérisé en ce qu'il comporte onze lignes ($L_0$ à $L_{10}$) et en ce que les optotypes de la onzième ligne présentent la plus faible fréquence spatiale et sont répartis sur une première ligne ($L_0$) et une onzième colonne ($C_{11}$).

11. Dispositif de détermination de la sensibilité visuelle aux contrastes chez l'homme du type comportant un matériel de test d'acuité visuelle et/ou de sensibilité aux contrastes,
    - un moyen de support du matériel de test muni d'une face frontale diffusante adaptée pour porter ledit test,
    - un moyen d'éclairement logé à l'intérieur du moyen de support et adapté pour éclairer uniformément selon une luminosité prédéterminée la face frontale dudit moyen de support et,
    - une feuille placée sur la face frontale du moyen de support, ladite feuille comportant une pluralité de lignes et de colonnes d'optotypes caractérisé en ce que
      * les optotypes d'une même fréquence spatiale sont rangés selon une même ligne $L_1$... $L_{10}$, ou respectivement colonne, ladite fréquence spatiale croissant de la première ligne $L_1$ à la dernière ligne $L_{10}$, en correspondance avec la fréquence spatiale utilisée pour des tests d'acuité,
      * les optotypes sont rangés selon des colonnes $C_1$-$C_{10}$, ou respectivement des lignes, constituant des ensembles d'isosensibilité aux contrastes, et dont le contraste de luminance présente des valeurs décroissant d'une première colonne $C_1$, ou respectivement ligne, présentant une valeur de contraste maximale, jusqu'à une dernière colonne $C_{10}$, ou respectivement ligne, présentant une valeur minimale de contraste.

12. Dispositif selon la revendication 11, caractérisé en ce que la dernière colonne $C_{10}$ présente les valeurs de contrastes de luminance suivantes, exprimées en pourcentage de contrastes par rapport au fond sur lequel les optotypes sont réalisés : pour les lignes $L_1$ et $L_2$ un contraste de 2,0 à 3,0 %, pour les lignes $L_3$ et $L_4$, 3,1 à 3,5 %, pour les lignes $L_5$ et $L_6$, 3,6 à 4,0 %, pour les lignes $L_7$ et $L_8$, 4,1 à 5,0 % et pour les lignes $L_9$ et $L_{10}$, 5,1 à 6,5 %.

13. Dispositif selon l'une des revendications 11 ou 12, caractérisé en ce que la feuille est translucide.

14. Dispositif selon l'une des revendications 11 à 13, caractérisé en ce qu'il comporte un moyen de sélection de la luminosité du moyen d'éclairement.

15. Dispositif selon la revendication 14, caractérisé en ce que le moyen de sélection est adapté pour permettre un choix entre au moins trois niveaux de luminosité distincts, un niveau de faible luminosité, un niveau de moyenne luminosité et un niveau de forte luminosité.

16. Dispositif selon la revendication 15, caractérisé en ce que le moyen de sélection est adapté pour faire correspondre respectivement aux niveaux de faible, moyenne et forte luminosité, des éclairements de la face diffusante du moyen de support de l'ordre de 3 à 10 $cd/m^2$, 80 à 90 $cd/m^2$, et 600 à 1 000 $cd/m^2$.

17. Dispositif selon l'une des revendications 14 à 16, caractérisé en ce que le moyen de sélection est un dispositif intégré dans le moyen de support.

18. Dispositif selon l'une des revendications 14 à 16, caractérisé en ce que le moyen de sélection est un dispositif à télécommande infrarouge.

**19.** Dispositif selon l'une des revendications 12 à 18, caractérisé en ce que le moyen d'éclairement comprend une pluralité de sources lumineuses uniformément réparties.

**20.** Dispositif selon la revendication 19, caractérisé en ce que ladite pluralité de sources lumineuses comprend au moins quatre tubes-néons.

**21.** Dispositif selon l'une des revendications 14 à 20, caractérisé en ce que le moyen de sélection est associé à une carte électronique adaptée pour commander l'intensité du courant délivré au moyen d'éclairement selon la luminosité choisie.

**22.** Procédé de fabrication d'un matériel de test d'acuité visuelle et/ou de sensibilité aux contrastes selon la revendication 1, caractérisé en ce qu'il consiste à :
- créer à l'aide d'une unité centrale de calcul des optotypes de grandeur déterminée afin de leur conférer une fréquence spatiale spécifique,
- donner aux optotypes de grandeur déterminée une densité déterminée afin de leur conférer une valeur de contraste spécifique,
- ranger tous les optotypes de même fréquence spatiale selon une même ligne ou respectivement selon une même colonne,
- ranger les optotypes selon des colonnes, ou respectivement selon des lignes, dont le contraste de luminance présente des valeurs décroissant d'une première colonne $C_1$, ou respectivement ligne, présentant une valeur maximale de contraste, jusqu'à une dernière colonne $C_{10}$, ou respectivement ligne, présentant une valeur minimale de contraste.
- afficher sur un écran associé à l'unité centrale le tableau d'optotypes ainsi créés,
- réaliser un positif de l'écran sur un film photosensible,
- réaliser un négatif du positif,
- tirer par un procédé photographique ledit négatif,
- vérifier le contraste de chacun des optotypes apparaissant sur le tirage,
- en déduire les temps d'exposition optimum du négatif pour obtenir les contrastes désirés et,
- tirer par un procédé photographique ledit négatif afin de réaliser une pluralité de matériels de tests de contraste identiques.

**23.** Procédé selon la revendication 22, caractérisé en ce que l'on vérifie le contraste de façon à obtenir une dernière colonne $C_{10}$ présentant les valeurs de contrastes de luminance suivantes, exprimées en pourcentage de contrastes par rapport au fond sur lequel les optotypes sont réalisés : pour les lignes $L_1$ et $L_2$ un contraste de 2,0 à 3,0 %, pour les lignes $L_3$ et $L_4$, 3,1 à 3,5 %, pour les lignes $L_5$ et $L_6$, 3,6 à 4,0 %, pour les lignes $L_7$ et $L_8$, 4,1 à 5,0 % et pour les lignes $L_9$ et $L_{10}$, 5,1 à 6,5 %.

**24.** Procédé selon l'une des revendications 22 ou 23, caractérisé en ce qu'on range la première ligne $L_1$ d'optotypes du tableau de sorte qu'elle comporte les optotypes de plus faible fréquence spatiale et les lignes ($L_2$ à $L_{10}$) suivantes par fréquences spatiales de plus en plus élevées.

**25.** Procédé selon l'une des revendications 22 à 24, caractérisé en ce qu'on effectue le tirage du matériel de test sur une feuille transparente ou translucide.

**Claims**

**1.** Equipment for testing visual acuity and/or visual sensitivity to spatial contrasts in humans, of the type comprising a sheet provided on one of its faces with a plurality of optotypes distributed in the form of rows and columns, characterised in that:
- the optotypes of the same spatial frequency are arranged in the same row $L_1$ ... $L_{10}$, or column, the said spatial frequency increasing from the first row $L_1$ to the last row $L_{10}$, corresponding with the spatial frequency used for acuity tests,
- the optotypes are arranged in columns $C_1$-$C_{10}$, or lines, constituting contrast isosensitivity sets, and the luminance contrast of which has values decreasing from a first column $C_1$, or row, with a maximum contrast value, to a last column $C_{10}$, or row, with a minimum contrast value.

**2.** Test equipment according to Claim 1, characterised in that the last column $C_{10}$ has the following luminance contrast values, expressed as a contrast percentage with respect to the background on which the optotypes are produced: for rows $L_1$ and $L_2$ a contrast of 2.0 to 3.0%, for rows $L_3$ and $L_4$, 3.1 to 3.5%, for rows $L_5$ and $L_6$, 3.6 to 4.0%, for rows $L_7$ and $L_8$, 4.1 to 5.0%, and for rows $L_9$ and $L_{10}$, 5.1 to 6.5%.

3. Test equipment according to one of Claims 1 or 2, characterised in that the contrast values decrease logarithmically from one column to the next.

4. Test equipment according to one of Claims 1 to 3, characterised in that it comprises 10 rows and 10 columns.

5. Test equipment according to one of Claims 1 to 4, characterised in that the spatial frequency of each of the rows $L_1$ to $L_{10}$ corresponds respectively to the following values: 3; 6; 9; 12; 15; 17.5; 20; 24; 27 and 30 cycles per degree.

6. Test equipment according to one of the preceding claims, characterised in that each of the rows $L_1$ to $L_{10}$ corresponds, when seen from a predetermined distance, respectively to the following degrees of visual acuity: 1/10; 2/10; 3/10; 4/10; 5/10; 6/10; 7/10; 8/10; 9/10; 1.

7. Test equipment according to one of the preceding claims, characterised in that the optotypes used are letters.

8. Test equipment according to one of Claims 1 to 6, characterised in that the optotypes used are images or figures designed so as to be recognisable by children or illiterates.

9. Test equipment according to one of the preceding claims, characterised in that the sheet is translucent.

10. Test equipment according to one of Claims 1, 2, 3, 7, 8, 9, characterised in that it comprises eleven rows ($L_0$ to $L_{10}$) and in that the optotypes on the eleventh row have the lowest spatial frequency and are distributed over a first row ($L_0$) and an eleventh column ($C_{11}$).

11. Device for determining human visual sensitivity to contrasts, of the type comprising equipment for testing visual acuity and/or sensitivity to contrasts
    - a means of supporting the test equipment having a diffusing front face adapted for carrying the said test,
    - an illumination means housed inside the support means and adapted for illuminating uniformly, according to a predetermined luminosity, the front face of the said support means, and
    - a sheet placed on the front face of the support means, the said sheet comprising a plurality of rows and columns of optotypes
    characterised in that
      . the optotypes of the same spatial frequency are arranged in the same row $L_1$ ... $L_{10}$, or column, the said spatial frequency increasing from the first row $L_1$ to the last row $L_{10}$, corresponding with the spatial frequency used for acuity tests,
      . the optotypes are arranged in columns $C_1$-$C_{10}$, or lines, constituting contrast isosensitivity sets, and the luminance contrast of which has values decreasing from a first column $C_1$, or line, with a maximum contrast value, to a last column $C_{10}$, or line, with a minimum contrast value.

12. Device according to Claim 11, characterised in that the last column $C_{10}$ has the following luminance contrast values, expressed as a contrast percentage with respect to the background on which the optotypes are produced: for rows $L_1$ and $L_2$ a contrast of 2.0 to 3.0%, for rows $L_3$ and $L_4$, 3.1 to 3.5%, for rows $L_5$ and $L_6$, 3.6 to 4.0%, for rows $L_7$ and $L_8$, 4.1 to 5.0%, and for rows $L_9$ and $L_{10}$, 5.1 to 6.5%.

13. Device according to one of Claims 11 or 12, characterised in that the sheet is translucent.

14. Device according to one of Claims 11 to 13, characterised in that it comprises a means of selecting the luminosity of the illumination means.

15. Device according to Claim 14, characterised in that the selection means is adapted for allowing a choice between at least three distinct luminosity levels, a low luminosity level, a medium luminosity level and a high luminosity level.

16. Device according to Claim 15, characterised in that the selection means is adapted for causing illuminations of the diffusing face of the support means of around 3 to 10 $cd/m^2$, 80 to 90 $cd/m^2$ and 600 to 1000 $cd/m^2$ to correspond respectively to the low, medium and high luminosity levels.

17. Device according to one of Claims 14 to 16, characterised in that the selection means is a device integrated into the support means.

18. Device according to one of Claims 14 to 16, characterised in that the selection means is an infrared remote-control device.

19. Device according to one of Claims 12 to 18, characterised in that the illumination means comprises a plurality of uniformly distributed light sources.

20. Device according to Claim 19, characterised in that the said plurality of light sources comprises at least four neon tubes.

21. Device according to one of Claims 14 to 20, characterised in that the selection means is associated with an electronic board adapted for controlling the intensity of the current delivered to the illumination means according to the luminosity selected.

22. Process for manufacturing equipment for testing visual acuity and/or sensitivity to contrasts according to Claim 1, characterised in that it consists of:
   - creating, by means of a central computing unit, optotypes of a size determined so as to confer on them a specific spatial frequency,
   - giving the optotypes of a given size a density determined so as to confer on them a specific contrast value,
   - arranging all the optotypes of the same spatial frequency in the same row or the same column,
   - arranging the optotypes in columns, or in lines, which have a luminance contrast with values decreasing from a first column $C_1$ or line respectively, with a maximum contrast value, to a last column $C_{10}$ or line respectively, with a minimum contrast value,
   - displaying the table of optotypes thus created on a screen associated with the central unit,
   - producing a positive of the screen on a photosensitive film,
   - producing a negative of the positive,
   - printing the said negative by means of a photographic process,
   - verifying the contrast of each of the optotypes appearing on the print,
   - deriving therefrom the optimum exposure times for the negative in order to obtain the required contrasts, and
   - printing the said negative by means of a photographic process in order to produce a plurality of identical items of contrast test equipment.

23. Process according to Claim 22, characterised in that the contrast is verified so as to obtain a last column $C_{10}$ having the following luminance contrast values, expressed as a contrast percentage with respect to the background on which the optotypes are produced: for rows $L_1$ and $L_2$ a contrast of 2.0 to 3.0%, for rows $L_3$ and $L_4$, 3.1 to 3.5%, for rows $L_5$ and $L_6$, 3.6 to 4.0%, for rows $L_7$ and $L_8$, 4.1 to 5.0%, and for rows $L_9$ and $L_{10}$, 5.1 to 6.5%.

24. Process according to Claim 22 or 23, characterised in that the first row $L_1$ of optotypes in the table is arranged so that it includes the optotypes with the lowest spatial frequency and the following rows ($L_2$ to $L_{10}$) are arranged in higher and higher spatial frequencies.

25. Process according to one of Claims 22 to 24, characterised in that the test equipment is printed on a transparent or translucent sheet.

**Patentansprüche**

1. Tafel zum Testen der Sehschärfe und/oder der visuellen Empfindlichkeit für Raumkontraste beim Menschen, der Art, die eine an einer ihrer Oberflächen mit einer Mehrzahl von Optotypen versehene Folie umfaßt, wobei die besagten Optotypen in der Form von Zeilen und Spalten verteilt sind, dadurch gekennzeichnet, daß:

   . die Optotypen einer gleichen Raumfrequenz in einer gleichen Zeile $L_1 ... L_{10}$ bzw. Spalte angeordnet sind, wobei die besagte Raumfrequenz von der ersten Zeile $L_1$ bis zu der letzten Zeile $L_{10}$ der für die Sehschärfetests verwendeten Raumfrequenz entsprechend zunimmt,

   . die Optotypen in Spalten $C_1$-$C_{10}$ bzw. Zeile angeordnet sind, die Kollektive gleicher Kontrastempfindlichkeit bilden und deren Leuchtdichtekontrast von einer ersten Spalte $C_1$ bzw. Zeile, in der der Kontrastwert maximal ist, bis zu einer letzten Spalte $C_{10}$ bzw. Zeile, in der der Kontrastwert minimal ist, abnimmt.

2. Testtafel nach Anspruch 1, dadurch gekennzeichnet, daß die letzte Spalte $C_{10}$ die folgenden Leuchtdichtekontrastwerte, ausgedrückt als prozentuale Kontrastanteile im Verhältnis zu der Grundlage, auf der die Optotypen ausgeführt sind, aufweist: hinsichtlich der Zeilen $L_1$ und $L_2$ einen Kontrast von 2,0 bis 3,0 %, hinsichtlich der Zeilen $L_3$ und $L_4$ 3,1 bis 3,5 %, hinsichtlich der Zeilen $L_5$ und $L_6$ 3,6 bis 4,0 %, hinsichtlich der Zeilen $L_7$ und $L_8$ 4,1 bis 5,0 % und hinsichtlich der Zeilen $L_9$ und $L_{10}$ 5,1 bis 6,5 %.

3. Testtafel nach einem der Ansprüche 1 oder 2, dadurch gekennzeichnet, daß die Kontrastwerte von Spalte zu Spalte auf logarithmische Weise abnehmen.

4. Testtafel nach einem der Ansprüche 1 bis 3, dadurch gekennzeichnet, daß sie 10 Zeilen und 10 Spalten umfaßt.

5. Testtafel nach einem der Ansprüche 1 bis 4, dadurch gekennzeichnet, daß die Raumfrequenz jeder der der Zeilen $L_1$ bis $L_{10}$ den folgenden Werten entspricht: 3 ; 6 ; 9 ; 12 ; 15 ; 17,5 ; 20 ; 24 ; 27 bzw. 30 Zyklen je Grad.

6. Testtafel nach einem der vorstehenden Ansprüche, dadurch gekennzeichnet, daß jede der Zeilen $L_1$ bis $L_{10}$ aus einer vorbestimmten Distanz betrachtet den folgenden Graden der Sehschärfe entspricht: 1/10 ; 2/10 ; 3/10 ; 4/10 ; 5/10 ; 6/10 ; 7/10 ; 8/10 ; 9/10 ; 1.

7. Testtafel nach einem der vorstehenden Ansprüche, dadurch gekennzeichnet, daß die verwendeten Optotypen Buchstaben sind.

8. Testtafel nach einem der Ansprüche 1 bis 6, dadurch gekennzeichnet, daß die verwendeten Optotypen Bilder oder Ziffern sind, die so gestaltet wurden, daß sie von Kindern oder Analphabeten erkannt werden können.

9. Testtafel nach einem der vorstehenden Ansprüche, dadurch gekennzeichnet, daß die Folie lichtdurchlässig ist.

10. Testtafel nach einem der Ansprüche 1, 2, 3, 7, 8, 9, dadurch gekennzeichnet, daß sie elf Zeilen ($L_0$ bis $L_{10}$) umfaßt, sowie dadurch, daß die Optotypen der elften Zeile die geringste Raumfrequenz aufweisen und in einer ersten Zeile ($L_0$) und einer elften Spalte ($C_{11}$) verteilt sind.

11. Vorrichtung zur Bestimmung der visuellen Empfindlichkeit für Kontraste beim Menschen, der Art, die eine Tafel zum Testen der Sehschärfe und/oder der Empfindlichkeit für Kontraste umfaßt, sowie

   - ein Mittel zum Abstützen der Testtafel, das mit einer vorderen Streufläche versehen ist, an der die besagte Testtafel angebracht werden kann,
   - ein Beleuchtungsmittel im Inneren des Abstützmittels, das so beschaffen ist, daß es die Vorderfläche des besagten Abstützmittels bei einer vorbestimmten Leuchtstärke gleichmäßig beleuchtet, und
   - eine an der Vorderfläche des Abstützmittels angeordnete Folie, wobei die besagte Folie eine Mehrzahl von Optotypzeilen und -spalten umfaßt, dadurch gekennzeichnet,

\* daß die Optotypen einer gleichen Raumfrequenz in einer gleichen Zeile $L_1$... $L_{10}$ bzw. Spalte angeordnet sind, wobei die besagte Raumfrequenz von der ersten Zeile $L_1$ bis zu der letzten Zeile $L_{10}$ im Einklang mit der für die Sehschärfetests verwendeten Raumfrequenz zunimmt,

\* wobei die Optotypen in Spalten $C_1$-$C_{10}$ bzw. Zeilen angeordnet sind, die Kollektive gleicher Empfindlichkeit für Kontraste bilden und bei denen der Leuchtdichtekontrast Werte aufweist, die von einer ersten Spalte $C_1$ bzw. einer Zeile, in der der Kontrastwert maximal ist, bis zu einer letzten Spalte $C_{10}$ bzw. Zeile, in der der Kontrastwert minimal ist, abnehmen.

12. Vorrichtung nach Anspruch 11, dadurch gekennzeichnet, daß die letzte Spalte $C_{10}$ die folgenden Leuchtdichtekontrastwerte, ausgedrückt als prozentuale Kontrastanteile im Verhältnis zu der Grundlage, auf der die Optotypen ausgeführt sind, aufweist: hinsichtlich der Zeilen $L_1$ und $L_2$ einen Kontrast von 2,0 bis 3,0 %, hinsichtlich der Zeilen $L_3$ und $L_4$ 3,1 bis 3,5 %, hinsichtlich der Zeilen $L_5$ und $L_6$ 3,6 bis 4,0 %, hinsichtlich der Zeilen $L_7$ und $L_8$ 4,1 bis 5,0 % und hinsichtlich der Zeilen $L_9$ und $L_{10}$ 5,1 bis 6,5 %.

13. Vorrichtung nach einem der Ansprüche 11 oder 12, dadurch gekennzeichnet, daß die Folie lichtdurchlässig ist.

14. Vorrichtung nach einem der Ansprüche 11 bis 13, dadurch gekennzeichnet, daß sie ein Mittel zum Wählen der Leuchtstärke des Beleuchtungsmittels umfaßt.

15. Vorrichtung nach Anspruch 14, dadurch gekennzeichnet, daß das Wählmittel so beschaffen ist, daß es die Wahl zwischen mindestens drei getrennten Leuchtstärkepegeln gestattet, und zwar einem Pegel geringer Leuchtstärke, einem Pegel mittlerer Leuchtstärke und einem Pegel hoher Leuchtstärke.

16. Vorrichtung nach Anspruch 15, dadurch gekennzeichnet, daß das Wählmittel so beschaffen ist, daß es den Pegeln geringer, mittlerer und hoher Leuchtstärke entsprechend Beleuchtungsstärken der Streufläche des Abstützmittels von etwa 3 bis 10 cd/m$^2$, 80 bis 90 cd/m$^2$ bzw. 600 bis 1000 cd/m$^2$ bedingt.

17. Vorrichtung nach einem der Ansprüche 14 bis 16, dadurch gekennzeichnet, daß das Wählmittel eine in dem Abstützmittel eingebaute Vorrichtung ist.

18. Vorrichtung nach einem der Ansprüche 14 bis 16, dadurch gekennzeichnet, daß das Wählmittel eine Vorrichtung mit Infrarot-Fernsteuerung ist.

19. Vorrichtung nach einem der Ansprüche 12 bis 18, dadurch gekennzeichnet, daß das Beleuchtungsmittel eine Mehrzahl von gleichmäßig verteilten Lichtquellen umfaßt.

20. Vorrichtung nach Anspruch 19, dadurch gekennzeichnet, daß die besagte Mehrzahl von Lichtquellen mindestens vier Neon-Röhren umfaßt.

21. Vorrichtung nach einem der Ansprüche 14 bis 20, dadurch gekennzeichnet, daß das Wählmittel mit einer elektronischen Leiterplatte in Verbindung steht, die so beschaffen ist, daß sie die Intensität des dem Beleuchtungsmittel zugeführten Stroms gemäß der gewählten Leuchtstärke steuert.

22. Verfahren zur Herstellung einer Tafel zum Testen der Sehschärfe und/oder Empfindlichkeit für Kontraste nach Anspruch 1, dadurch gekennzeichnet, daß sie darin besteht:
   - mit Hilfe einer zentralen Recheneinheit Optotypen vorgegebener Größe zu schaffen, um ihnen eine spezifische Raumfrequenz zu vermitteln,
   - Optotypen vorgegebener Größe eine bestimmte Dichte zu verleihen, um ihnen einen spezifischen Kontrastwert zu vermitteln,
   - alle Optotypen der gleichen Raumfrequenz in einer gleichen Zeile bzw. einer gleichen Spalte anzuordnen,
   - die Optotypen in Spalten bzw. Zeilen anzuordnen, bei denen der Leuchtdichtekontrast Werte aufweist, die von einer ersten Spalte $C_1$ bzw. Zeile, in der der Kontrastwert maximal ist, bis zu einer letzten Spalte $C_{10}$ bzw. Zeile, in der der Kontrastwert minimal ist, abnehmen.
   - auf einem mit der Zentraleinheit in Verbindung stehenden Bildschirm die Tafel der auf diese Weise geschaffenen Optotypen darzustellen,

- auf einem lichtempfindlichen Film ein Positiv des Bildschirms zu erzeugen,
- ein Negativ des Positivs zu erzeugen,
- das besagte Negativ nach einem fotografischen Verfahren abzudrucken,
- den Kontrast jedes der in dem Abdruck erscheinenden Optotypen zu überprüfen,
- daraus die zur Erzielung der gewünschten Kontraste erforderlichen optimalen Negativbelichtungszeiten abzuleiten, und
- das besagte Negativ nach einem fotografischen Verfahren abzudrucken, um eine Mehrzahl von identischen Tafeln zur Prüfung des Kontrastes herzustellen.

23. Verfahren nach Anspruch 22, dadurch gekennzeichnet, daß der Kontrast auf die Weise überprüft wird, daß man eine letzte Spalte $C_{10}$ erhält, die die folgenden Leuchtdichtekontrastwerte, ausgedrückt als prozentuale Anteile der Kontraste im Verhältnis zu der Grundlage, auf der die Optotypen ausgeführt sind, aufweist: hinsichtlich der Zeilen $L_1$ und $L_2$ einen Kontrast von 2,0 bis 3,0 %, hinsichtlich der Zeilen $L_3$ und $L_4$ 3,1 bis 3,5 %, hinsichtlich der Zeilen $L_5$ und $L_6$ 3,6 bis 4,0 %, hinsichtlich der Zeilen $L_7$ und $L_8$ 4,1 bis 5,0 % und hinsichtlich der Zeilen $L_9$ und $L_{10}$ 5,1 bis 6,5 %.

24. Verfahren nach einem der Ansprüche 22 oder 23, dadurch gekennzeichnet, daß die erste Zeile $L_1$ der Tafeloptotypen so angeordnet wird, daß sie die Optotypen der niedrigsten Raumfrequenz umfaßt, während die nachstehenden Zeilen ($L_2$ bis $L_{10}$) durch in zunehmendem Maße erhöhte Raumfrequenzen gekennzeichnet sind.

25. Verfahren nach einem der Ansprüche 22 bis 24, dadurch gekennzeichnet, daß der Abdruck der Testtafel auf einer durchsichtigen oder lichtdurchlässigen Folie bewirkt wird.

Fig.1

Fig. 2

Fig. 3

Fig. 4